# EUROPEAN PATENT APPLICATION

(11) **EP 1 437 142 A1**
(43) Date of publication of application: **14.07.2004**
(21) Application number: 02779913.9
(22) Date of filing: 16.10.2002
(51) Int. Cl.: A61K 45/00, A61K 31/445, A61P 35/00

(54) **CELL GROWTH INHIBITORS**

(30) Priority: 16.10.2001 JP 2001318439
(71) Applicant: CHUGAI SEIYAKU KABUSHIKI KAISHA, Tokyo, 115-8543 (JP)
(72) Inventor: SAITOH, Ryoichi CHUGAI SEIYAKU KABUSHIKI KAISHA, Gotenba-shi, Shizuoka 412-8513 (JP)
(74) Representative: VOSSIUS & PARTNER
(86) International application number: PCT/JP2002/010743
(87) International publication number: WO 2003/033024

(57) **Abstract**

The present invention revealed that AT-264 suppresses cell growth through its inhibitory effect on PepTl activity. Furthermore, as a result of examining whether AT-264 comprises the effect of inhibiting the cell growth of human pancreatic cancer cell line AsPC-1, the present invention revealed that cell growth is similarly suppressed. These findings show that cell growth can be suppressed by inhibiting the activity of peptide transporters. Suppression of peptide transporter activity can be considered to be an important indicator in the development of growth inhibitors for cancer cells and such.

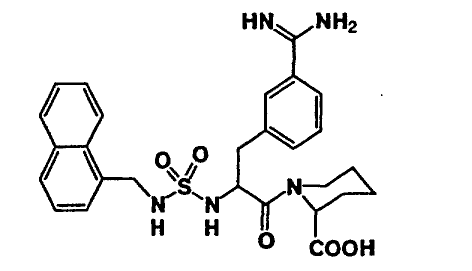

## Description

### Technical Field

The present invention relates to cell growth inhibitors comprising a peptide transporter inhibitory substance as an active ingredient.

### Background Art

Mammalian animals need to take in external sources of nutrition, and many transport proteins are known to exist in cells. Many peptide transporters (peptide transport proteins), which carry out peptide transport, have been found to date (for example, J. Biol. Chem., 270(12):6456-6463, (1995); Biochim. Biophys. Acta., 1235:461-466,(1995); Mol. Microbiol., Vol. 16, p825, (1995); Unexamined Published Japanese Patent Application No. (JP-A) Hei 6-261761; JP-A Hei 11-172; and US 5849525) . Peptide transporters can be classified into proteins that import peptides into cells, and proteins that export peptides from cells. They can also be classified according to the different energy sources used during transport. Proton-driven peptide transporters, which carry out transport by utilizing the difference between proton concentration inside and outside of a cell, belong to the PTR family (Mol. Microbiol., Vol. 16, p825, (1995)). Peptide transporters that carry out transport using ATP in the body belong to the ABC family (Annu. Rev. Cell. Biol., Vol. 8, p67, (1992)).

There are reports that peptide transporters are involved in the transport of not only small-molecule peptides such as dipeptides and tripeptides, but also of pharmaceutical agents such as β-lactam antibiotics and ACE inhibitors (Ganapathy, Leibach., Curr. Biol. 3, 695-701, (1991); Nakashima *et al.,* Biochem. Pharm. 33, 3345-3352, (1984); Friedman, Amidon., Pharm. Res., 6, 1043-1047, (1989); Okano *et al.,* J. Biol. Chem., 261, 14130-14134, (1986); Muranushi *et al.,* Pharm. Res., 6, 308-312, (1989); Friedman, Amidon., J. Control. Rel., 13, 141-146, (1990)).

PepT1 and PepT2 are proton-driven peptide transporters that contribute to the absorption of proteins and the maintenance of peptidic nitrogen sources by transporting small-molecule peptides into cells. PepT1 and PepT2 are 12-transmembrane proteins, comprising 708 and 729 amino acids, respectively (J. Biol. Chem., 270(12):6456-6463, (1995); Biochim. Biophys. Acta., 1235:461-466, (1995); and Terada and Inui, Tanpakusitsu Kakusan Kouso., Vol. 46, No. 5, (2001)).

There are reports that PepT1 and PepT2 also transport pharmaceuticals such as β-lactam antibiotics and bestatin (Saito, H. *et al.*, J. Pharmacol. Exp. Ther., 275, 1631-1637, (1995); Saito, H. *et al.*, Biochim. Biophys. Acta., 1280, 173-177, (1996); and Terada, T. *et al.*, J. Pharmacol. Exp. Ther., 281, 1415-1421 (1997)).

PepT1 is mainly expressed in the small intestine, and its expression has also been confirmed in the kidney and pancreas. Expression of PepT2 has been confirmed in the kidney, brain, lung, and spleen. PepT1 and PepT2 have been reported to be localized in the small intestine, and in the brush border membrane of renal tubular epithelial cells (Ogihara, H. *et al.*, Biochem. Biophys. Res. Commun. 220, 848-852, (1996) ; Takahashi, K. *et al.,* J. Pharmacol. Exp. Ther., 286, 1037-1042 (1998); Hong, S. *et al.,* Am. J. Physiol. Renal. Physiol., 276, F658-F665 (1999); and Terada and Inui, Tanpakusitsu Kakusan Kouso., Vol. 46, No. 5, (2001)).

Furthermore, overexpression of PepT1 in the cell membrane of human pancreatic duct carcinoma cell lines (Cancer Res., 58, 519-525, (1998)), and expression of PepT2 mRNA in human pancreatic duct carcinoma cell lines (Millennium World Congress of Pharmaceutical Sciences, (2000)) have been reported. However, the involvement of PepT1 and PepT2 in cancer cell growth was unclear, and no discussion had been made as to whether inhibiting the function of PepT1 and PepT2 will affect cancer cell proliferation.

### Disclosure of the Invention

Such circumstances in the art led to the present invention, the objective of which is to provide cell growth inhibitors comprising a peptide transporter inhibitory substance as an active ingredient, and specifically, to provide cell growth inhibitors for cancers such as pancreatic cancer.

The present inventors found that AT-264 suppresses cell growth through its inhibitory effect on PepT1 activity. Furthermore, as a result of examining whether AT-264 comprises the effect of inhibiting the cell growth of human pancreatic cancer cell line AsPC-1, the present inventors found that it suppressed cell growth in the same way. These findings show that cell growth can be suppressed by inhibiting the activity of peptide transporters. Suppression of peptide transporter activity can be considered as an important indicator for the development of growth inhibitors for cancer cells and such.

More specifically, the present invention provides:
[1] a cell growth inhibitor comprising as an active ingredient a substance that inhibits a peptide transporter;
[2] the cell growth inhibitor of [1], wherein the peptide transporter is a proton-driven peptide transporter;
[3] the cell growth inhibitor of [1], wherein the peptide transporter is PepT1 or PepT2;
[4] the cell growth inhibitor of any one of [1] to [3], wherein the peptide transporter inhibitory substance is a substance that inhibits the transport function of a peptide transporter by binding to the peptide transporter;
[5] the cell growth inhibitor of any one of [1] to [3], wherein the peptide transporter inhibitory substance is a sulfamide derivative represented by the following formula (I): 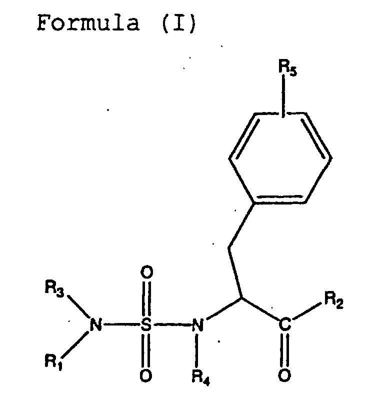 wherein,
   R₁ represents a hydrogen atom, lower alkyl group, or amino protecting group;
   R₂ represents a substituted or unsubstituted, fused or non-fused nitrogen atom-containing heterocycle;
   R₃ represents a A-(CH₂)ₘ- group, a hydrogen atom, or a substituted or unsubstituted lower alkyl group, wherein, A represents a substituted or unsubstituted aryl group, substituted or unsubstituted, fused or non-fused heterocycle, or substituted or unsubstituted lower cycloalkyl group, and m represents an integer from 0 to 6, and the - (CH₂)ₘ- may be substituted with one or more substituents;
   R₄ represents a hydrogen atom, lower alkyl group, or amino protecting group;
   R₅ represents -C (=NR₆) NH₂, -NH-C(=NR₆)NH₂, or - (CH₂)ₙ-NHR₆ group,
   wherein R₆ represents a hydrogen atom, lower alkyl group, hydroxyl group, acyl group, acyloxy group, lower alkoxy group, lower alkoxycarbonyl group, lower alkoxycarbonyloxy group, or lower hydroxyalkylcarbonyloxy group, n represents an integer from 0 to 2, and the -(CH₂)ₙ- may be substituted with one or more substituents;
[6] the cell growth inhibitor of any one of [1] to [5], wherein the inhibitor suppresses the growth of a cancer cell; and
[7] the cell growth inhibitor of [6], wherein the cancer cell is a pancreatic cancer cell.

The present invention provides cell growth inhibitors comprising a peptide transporter inhibitory substance as an active ingredient. Peptide transporters targeted by the cell growth inhibitors of this invention are not particularly limited, however are preferably peptide transporters which incorporate peptides into cells using proton motive force. More preferably, they are PepT1 or PepT2, and most preferably, they are PepT1.

The nucleotide and amino acid sequences of PepT1 and PepT2 are already known (human PepT1: GenBank XM_007063 (J. Biol. Chem., 270(12):6456-6463, (1995)); and human PepT2: GenBank XM_002922 (Biochim. Biophys. Acta., 1235:461-466, (1995))).

There are no limitations as to the peptide transporter inhibitory substances of the present invention, as long as they inhibit peptide transporter-mediated transport, and suppress cell growth. Examples of substances that inhibit peptide transporter-mediated transport include substances that inhibit the transport function of a peptide transporter by binding thereto, and substances that suppress peptide transporter expression. Substances that inhibit the transport function of peptide transporters by binding thereto are preferred.

Examples of substances that bind to peptide transporters include synthetic low-molecular-weight compounds, antibodies, proteins, peptides, and natural compounds. The compounds represented by the formula (1) below (see, WO 97/19919) or antibodies are preferred.
Sulfamide derivatives represented by formula (1) 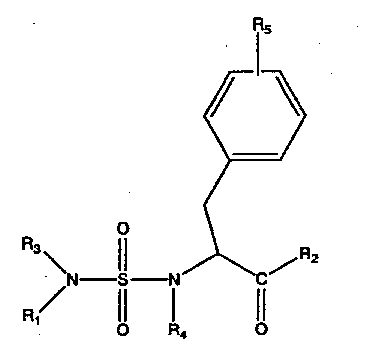 wherein,
R₁ represents a hydrogen atom, lower alkyl group, or amino protecting group;
R₂ represents a substituted or unsubstituted, fused or non-fused nitrogen-atom-containing heterocycle;
R₃ represents a A-(CH₂)ₘ- group, hydrogen atom, or substituted or unsubstituted lower alkyl group, wherein, A represents a substituted or unsubstituted aryl group, substituted or unsubstituted, fused or non-fused heterocycle, or substituted or unsubstituted lower cycloalkyl group, m represents an integer of 0 to 6, and the - (CH₂)ₘ-may be substituted with one or more substituents;
R₄ represents a hydrogen atom, lower alkyl group, or amino protecting group;
R₅ represents -C(=NR₆)NH₂, -NH-C(=NR₆)NH₂, or -(CH₂)ₙ-NHR₆ group,
wherein R₆ represents a hydrogen atom, lower alkyl group, hydroxyl group, acyl group, acyloxy group, lower alkoxy group, lower alkoxycarbonyl group, lower alkoxycarbonyloxy group, or lower hydroxyalkylcarbonyloxy group, n represents an integer of 0 to 2, and the -(CH₂)ₙ- may be substituted with one or more substituents.

In the present invention, the following terms have the following meanings, unless there are particular limitations.

The lower alkyl group means a linear or branched alkyl group, wherein the number of carbons ranges from one to six, and preferably from one to four. Examples include a methyl group, ethyl group, n-propyl group, i-propyl group, n-butyl group, i-butyl group, s-butyl group, and t-butyl group.

The lower alkoxy group means a linear or branched alkyloxy group, wherein the number of carbons ranges from one to six, preferably ranges from one to four. Examples include a methoxy group, ethoxy group, n-propoxy group, i-propoxy group, n-butoxy group, i-butoxy group, s-butoxy group, and t-butoxy group.

The amino protecting group refers to any group that can protect the amino group to which R₁ is bound in the process of synthesizing a compound of formula (1) , and conventionally usable amino protecting groups can be used. Examples of such amino protecting groups include a formyl group, acetyl group, benzoyl group, trifluoroacetyl group, benzyloxycarbonyl group, methoxycarbonyl group, t-butoxycarbonyl group, phthaloyl group, benzyl group, and tosyl group, and preferably a t-butoxycarbonyl group.

Furthermore, the substituted or unsubstituted amino group means an amino group that may be substituted with one or more substituents, such as the aforementioned amino protecting group, hydroxyl group, substituted or unsubstituted lower alkyl group, substituted or unsubstituted acyl group including a substituted or unsubstituted lower alkoxycarbonyl group or a substituted or unsubstituted lower alkylaminocarbonyl group, substituted or unsubstituted aryl group, substituted or unsubstituted sulfonyl group, substituted or unsubstituted, fused or non-fused heterocyclic group, substituted or unsubstituted lower alkoxy group, substituted or unsubstituted cycloalkyl group, substituted or unsubstituted cycloalkyloxy group, substituted or unsubstituted aryloxy group, substituted or unsubstituted, fused or non-fused heterocyclic-oxy group, and a substituted or unsubstituted silyl group. Examples include a methylamino group, ethylamino group, acetylamino group, dimethylaminocarbonylamino group, phenylamino group, p-toluenesulfonylamino group, methanesulfonylamino group, 4-piperidinylamino group, cyclohexylamino group, cyclopentylamino group, and cyclopropylamino group, and preferably include a methylamino group, ethylamino group, acetylamino group, p-toluenesulfonylamino group, methanesulfonylamino group, and cyclopropylamino group.

The substituted or unsubstituted lower alkyl group means a lower alkyl group that may be substituted with one or more substituents such as a halogen atom, hydroxyl group, thiol group, substituted or unsubstituted amino group, substituted or unsubstituted acyl group such as a substituted or unsubstituted lower alkoxycarbonyl group or a substituted or unsubstituted lower alkylaminocarbonyl group, nitro group, cyano group, substituted or unsubstituted aryl group, substituted or unsubstituted sulfonyl group, substituted or unsubstituted, fused or non-fused heterocyclic group, substituted or unsubstituted carboxyl group, substituted or unsubstituted lower alkoxy group, substituted or unsubstituted cycloalkyl group, substituted or unsubstituted cycloalkyloxy group, substituted or unsubstituted aryloxy group, substituted or unsubstituted lower alkylthio group, substituted or unsubstituted, fused or non-fused heterocyclic-oxy group, substituted or unsubstituted cycloalkylthio group, substituted or unsubstituted, fused or non-fused heterocyclic-thio group, substituted or unsubstituted arylthio group, substituted or unsubstituted sulfonyloxy group, and substituted or unsubstituted silyl group. Examples include a 2-(pyrrolidine-1-ylcarbonyl)ethyl group, 3-phenyl-2-(pyrrolidine-1-ylcarbonyl)-n-propyl group, 3,3-diphenyl-n-propyl group, 2,2-diphenylethyl group, and 2-cyclohexyloxyethyl group, and preferably include a 3-phenyl-2-(pyrrolidine-1-ylcarbonyl)-n-propyl group, 3,3-diphenyl-n-propyl group, and 2,2-diphenylethyl group.

The substituted or unsubstituted lower alkoxy group means a lower alkoxy group substituted with substituents similar to those described for the above-mentioned lower alkyl groups. Examples include a fluoromethoxy group, fluoroethoxy group, and benzyloxy group.

The aryl group is a group in which one hydrogen atom is removed from an aromatic hydrocarbon. Examples include a phenyl group, tolyl group, naphthyl group, xylyl group, biphenyl group, anthryl group and phenanthryl group, and preferably include a phenyl group and naphthyl group.

The substituted or unsubstituted aryl group means the abovementioned aryl group in which an arbitrary hydrogen atom may be substituted with one or more substituents, including a substituted or unsubstituted lower alkyl group, substituted or unsubstituted lower alkoxy group, halogen atom, hydroxyl group, thiol group, substituted or unsubstituted amino group, substituted or unsubstituted acyl group, substituted or unsubstituted lower alkylthio group, nitro group, cyano group, substituted or unsubstituted aryl group, substituted or unsubstituted arylalkyl group, substituted or unsubstituted aryloxy group, substituted or unsubstituted sulfonyl group, substituted or unsubstituted carboxyl group, substituted or unsubstituted lower alkylsulfonyl group, substituted or unsubstituted lower alkylsulfonylamino group, substituted or unsubstituted, fused or non-fused heterocyclic group, substituted or unsubstituted cycloalkylthio group, substituted or unsubstituted sulfonyloxy group, substituted or unsubstituted arylthio group, substituted or unsubstituted silyl group, substituted or unsubstituted, fused or non-fused heterocyclic-oxy group, and a substituted or unsubstituted, fused or non-fused heterocyclic-thio group. Examples include an o-methylphenyl group, m-hydroxyphenyl group, p-carboxylpheyl group, 2-phenethylphenyl group, 2,3-dimethoxyphenyl group, 2-methyl-4-aminophenyl group, phenoxyphenyl group, 3-phenethylphenyl group, 5-cyanonaphthyl group, 4-amino-1-naphthyl group, 6-hydroxy-1-naphthyl group, 3-methoxyphenyl group, 2-methoxyphenyl group, 2-ethoxyphenyl group, 2-benzylphenyl group, 3-bromo-1-naphthyl group, 6-methoxy-1-naphthyl group, 1-naphthyl group, and 2-naphthyl group, and preferably include a 2-phenethylphenyl group, 6-hydroxy-1-naphthyl group, 3-bromo-1-naphthyl group, and 2,3-dimethoxyphenyl group. A substituted or unsubstituted cycloalkyl group means a cycloalkyl group with three to seven carbons, preferably four to six carbons, of which an arbitrary hydrogen atom may be substituted with one or more substituents, such as groups similar to those of the aforementioned aryl group. Examples include a cyclopropyl group, cyclobutyl group, cyclopentyl group, cyclohexyl group, 1-fluorocyclopropyl group, 2-benzylcyclohexyl group, 2-aminocyclopentyl group, 2-carboxycyclopentyl group, and 2-(6-methoxy-1,4-benzoquinone), and preferably a cyclohexyl group.

The substituted or unsubstituted, fused or non-fused nitrogen atom-containing heterocycle means a saturated or unsaturated three-to seven-membered heterocycle which contains one or more nitrogen atoms as a heteroatom, and which may further contain heteroatoms such as oxygen atoms and sulfur atoms. The nitrogen atom-containing heterocycle may be fused to one or more three- to seven-membered aromatic rings, heterocycles, or cycloalkyl rings. An arbitrary hydrogen atom bound to a ring carbon atom may be substituted with one or more substituents, such as those similar to the substituents of the aforementioned aryl group. Examples of a nitrogen atom-containing heterocycle include an aziridine ring, azetidine ring, pyrrole ring, pyrroline ring, pyrrolidine ring, indole ring, indoline ring, isoindole ring, octahydroindole ring, carbazole ring, pyridine ring, piperidine ring, quinoline ring, dihydroquinoline ring, tetrahydroquinoline ring, decahydroquinoline ring, isoquinoline ring, tetrahydroisoquinoline ring, decahydroisoquinoline ring, quinolone ring, acridine ring, phenanthridine ring, benzoquinoline ring, pyrazole ring, imidazole ring, imidazoline ring, imidazolidine ring, benzoimidazole ring, pyridazine ring, pyrimidine ring, pyrazine ring, piperazine ring, benzodiazine ring, triazole ring, benzotriazole ring, triazine ring, tetrazole ring, tetrazine ring, purine ring, xanthine ring, theophilline ring, guanine ring, pteridine ring, naphthylidine ring, quinolizine ring, quinuclidine ring, indolizine ring, oxazole ring, benzoxazole ring, isoxazole ring, oxazine ring, phenoxazine ring, thiazole ring, thiazolidine ring, benzothiazole ring, isothiazole ring, thiazine ring, oxadiazole ring, oxadiazine ring, thiadiazole ring, thiadiazine ring, dithiazine ring, and morpholine ring, and preferably include a piperidine ring, piperazine ring, isoquinoline ring, and tetrahydroisoquinoline ring. Preferred examples of those having substituents include an N-acetylpiperazine ring, N-p-toluenesulfonylpiperazine ring, and 4-methylpiperidine ring.

The substituted or unsubstituted, fused or non-fused heterocycle means a saturated or unsaturated three- to seven-membered heterocycle containing one or more nitrogen atoms, oxygen atoms, or sulfur atoms as a heteroatom. The heterocycle may be fused to one or more three- to seven-membered aromatic rings, heterocycles, and cycloalkyl rings. An arbitrary hydrogen atom bound to a ring carbon atom may be substituted with one or more substituents such as those similar to the substituents of the aforementioned aryl group. Besides the aforementioned nitrogen atom-containing heterocycle, examples of such a heterocycle include a pyran ring, furan ring, tetrahydropyran ring, tetrahydrofuran ring, thiophene ring, benzothiophene ring, dihydrobenzothiophene ring, benzofuran ring, isobenzofuran ring, chroman ring, chromene ring, dibenzofuran ring, isochroman ring, phenoxatine ring, xanthine ring, thianthrene ring, benzodioxane ring, benzodioxolane ring, and thiolane ring, and preferably a benzothiophene ring.

The acyl group is a group in which the OH in the carboxyl group of a carboxylic acid has been removed. Examples include a formyl group, acetyl group, propionyl group, butyryl group, valeryl group, oxalyl group, malonyl group, succinyl group, benzoyl group, toluoyl group, naphthoyl group, phthaloyl group, pyrolidinecarbonyl group, and pyridinecarbonyl group, and preferably include an acetyl group and benzoyl group. Furthermore, the substituted or unsubstituted acyl group means an acyl group substituted with a lower alkyl group, or another group similar to those shown for the aforementioned lower alkyl group, as a substituent. Examples include a substituted or unsubstituted lower alkylcarbonyl group, substituted or unsubstituted lower alkylaminocarbonyl group, substituted or unsubstituted lower alkyloxycarbonyl group, and aminocarbonylcarbonyl group.

The acyloxy group means an acyl group to which an oxygen atom is bound, and examples include an acetoxy group and benzoyloxy group.

The lower alkoxycarbonyl group means a lower alkoxy group to which a carbonyl group is bound, in which the number of carbons in the alkoxy portion ranges from one to six, and preferably ranges from one to four. Examples include a methoxycarbonyl group, ethoxycarbonyl group, n-propoxycarbonyl group, i-propoxycarbonyl group, n-butoxycarbonyl group, i-butoxycarbonyl group, s-butoxycarbonyl group, and t-butoxycarbonyl group, and preferably include a methoxycarbonyl group and ethoxycarbonyl group.

The lower alkoxycarbonyloxy group means a lower alkoxycarbonyl group to which an oxygen atom is bound, in which the number of carbons in the alkoxy portion ranges from one to six, and preferably ranges from one to four. Examples include a methoxycarbonyloxy group, ethoxycarboxyloxy group, n-propoxycarbonyloxy group, i-propoxycarbonyloxy group, n-butoxycarbonyloxy group, i-butoxycarbonyloxy group, s-butoxycarbonyloxy group, and t-butoxycarbonyloxy group, and preferably include a methoxycarbonyloxy group and ethoxycarbonyloxy group. Hydroxyalkylcarbonyloxy group refers to the aforementioned lower alkyl group substituted with one or more hydroxyl groups to which a carbonyloxy group (COO) is bound. Examples include a hydroxymethylcarbonyloxy group, 2-hydroxyethylcarbonyloxy group, and 2,3-dihydroxypropylcarbonyloxy group, in which the number of carbons in the alkyl portion ranges from one to six, and preferably ranges from one to four.

Examples of the halogen atom include a fluorine atom, chlorine atom, bromine atom, and iodine atom.

The lower alkylsulfonyl group is the aforementioned lower alkyl group to which a sulfonyl group is bound, in which the number of carbons ranges from one to six, and preferably ranges from one to four. Examples include a methylsulfonyl group, ethylsulfonyl group, n-propylsulfonyl group, and i-propylsulfonyl group.

Furthermore, an arylsulfonyl group means the aforementioned aryl group to which a sulfonyl group is bound, and preferably includes a phenylsulfonyl group and a naphthylsulfonyl group.

The substituted or unsubstituted lower alkylsulfonyl group and the substituted or unsubstituted arylsulfonyl group represent the aforementioned lower alkylsulfonyl group and arylsufonyl group in which an arbitrary hydrogen atom bound to a carbon atom thereof may be substituted with one or more substituents. Examples of the substituents include those similar to the substituents indicated for the aforementioned aryl group. Such examples include a p-toluenesulfonyl group and a trifluoromethanesulfonyl group.

The substituted or unsubstituted aminosulfonyl group is the aforementioned substituted or unsubstituted amino group to which a sulfonyl group is bound. Examples include a methylaminosulfonyl group and a benzylaminosulfonyl group.

The substituted or unsubstituted lower alkoxysulfonyl group means the aforementioned substituted or unsubstituted lower alkoxy group to which a sulfonyl group is bound, and preferably includes methoxysulfonyl group and benzyloxysulfonyl group. The substituted or unsubstituted cycloalkyloxysulfonyl group means a substituted or unsubstituted cycloalkyl group to which a sulfonyl group is bound through an oxygen atom. Examples include a cyclohexyloxysulfonyl group and cyclopentyloxysulfonyl group.

The substituted or unsubstituted cycloalkylsulfonyl group is the aforementioned substituted or unsubstituted cycloalkyl group to which a sulfonyl group is bound. Examples include a cyclohexylsulfonyl group and cyclopentylsulfonyl group.

The substituted or unsubstituted, fused or non-fused heterocyclic sulfonyl group means a substituted or unsubstituted heterocyclic group to which a sulfonyl group is bound, and preferably includes a 4-quinolylsulfonyl group and 8-tetrahydroquinolylsulfonyl group.

Furthermore, the substituted or unsubstituted sulfonyl group refers to a substituted or unsubstituted lower alkylsulfonyl group, substituted or unsubstituted cycloalkylsulfonyl group, substituted or unsubstituted cycloalkyloxysulfonyl group, substituted or unsubstituted aminosulfonyl group, substituted or unsubstituted, fused or non-fused heterocyclic sulfonyl group, substituted or unsubstituted lower alkoxysulfonyl group, or substituted or unsubstituted arylsulfonyl group.

The substituted or unsubstituted carboxyl group means the aforementioned substituted or unsubstituted acyl group to which an oxy group is bound. Examples include a methylcarbonyloxy group, ethylcarbonyloxy group, isopropylcarbonyloxy group, phenylcarbonyloxy group, and cyclohexylcarbonyloxy group.

The lower alkoxyalkyl group means the aforementioned lower alkoxy group to which a lower alkyl group is bound. Examples include a methoxymethyl group, methoxyethyl group, t-butoxymethyl group, 1-ethoxyethyl group, and 1-(isopropoxy)ethyl group. Furthermore, the alkoxy group or alkyl group portions of the lower alkoxylalkyl group may be substituted with groups similar to the substituents indicated for the aforementioned alkyl group.

The lower hydroxyalkyl group means the aforementioned lower alkyl group which is substituted with one or more hydroxyl groups. Examples include a hydroxymethyl group, 2-hydroxyethyl group, 1-hydroxyethyl group, 3-hydroxy-n-propyl group, and 2,3-dihydroxy-n-butyl group. Furthermore, the alkyl group portion of the lower hydroxyalkyl group may be substituted with groups similar to the substituents indicated for the aforementioned alkyl group.

The lower aminoalkyl group means the aforementioned substituted or unsubstituted amino group to which the aforementioned lower alkyl group is bound. Examples include a t-butylaminomethyl group, aminomethyl group, 2-aminoethyl group, benzylaminomethyl group, methylaminomethyl group, and 2-methylaminoethyl group. Furthermore, the alkyl group portion of the lower aminoalkyl group may be substituted with groups similar to the substituents indicated for the aforementioned alkyl group.

The lower carboxylalkyl group is the aforementioned substituted or unsubstituted carboxyl group to which the aforementioned lower alkyl group is bound. Examples include an acetyloxymethyl group, 2-acetyloxyethyl group, ethylcarbonyloxymethyl group, cyclohexylcarbonyloxymethyl group, cyclopropylcarbonyloxymethyl group, and isopropylcarbonyloxymethyl group. Furthermore, the alkyl group portion of the lower carboxylalkyl group may be substituted with groups similar to the substituents indicated for the aforementioned alkyl group.

The lower carbonylaminoalkyl group means the aforementioned substituted or unsubstituted acyl group to which the aforementioned lower aminoalkyl group is bound. Examples include an acetylaminomethyl group, t-butyloxycarbonylaminomethyl group, ethylcarbonylaminomethyl group, acetylaminoethyl group, and benzyloxycarbonylaminoethyl group. Furthermore, the amino group or alkyl group portion of the lower carbonylamino alkyl group may be substituted with groups similar to the substituents indicated for the aforementioned alkyl group.

The substituted or unsubstituted lower alkylthio group is the aforementioned substituted or unsubstituted lower alkyl group to which a thio group is bound. Examples include a methylthio group, ethylthio group, isopropylthio group, and t-butylthio group.

The substituted or unsubstituted cycloalkylthio group means the aforementioned substituted or unsubstituted cycloalkyl group to which a thio group is bound. Examples include a cyclopropylthio group, cyclobutylthio group, cyclopentylthio group, and cyclohexylthio group.

The substituted or unsubstituted arylthio group is the aforementioned substituted or unsubstituted aryl group to which a thio group is bound. Examples include a phenylthio group, 1-naphthylthio group, and 2-naphthylthio group.

The substituted or unsubstituted, fused or non-fused heterocyclic thio group means the aforementioned substituted or unsubstituted, fused or non-fused heterocyclic group to which a thio group is bound. Examples include a 4-quinolylthio group and 8-tetrahydroquinolylthio group.

The substituted or unsubstituted sulfonyloxy group is the aforementioned substituted or unsubstituted sulfonyl group to which an oxy group is bound. Examples include a p-toluenesulfonyloxy group and a methanesulfonyloxy group.

The substituted or unsubstituted cycloalkyloxy group is the aforementioned substituted or unsubstituted cycloalkyl group to which an oxy group is bound. Examples include a cyclopropyloxy group, cyclobentyloxy group, and 4-aminocyclohexyloxy group.

The substituted or unsubstituted, fused or non-fused heterocyclic-oxy group means the aforementioned substituted or unsubstituted, fused or non-fused heterocyclic group to which an oxy group is bound. Examples include a 4-quinolyloxy group and an 8-tetrahydroquinolyloxy group.

The substituted or unsubstituted silyl group refers to a silyl group to which one to three of the same or different aforementioned substituted or unsubstituted lower alkyl group or substituted or unsubstituted aryl group are bound. Examples include a trimethylsilyl group, triethylsilyl group, t-butyldimethylsilyl group, t-butyldiphenylsilyl group, and triisopropylsilyl group.

Furthermore, substituents that may be substituted in the - (CH₂)ₘ- portion and -(CH₂)ₙ- portion include substituents similar to those described as substituents of the aforementioned aryl group.

There are no particular limitations on the antibodies used as the active ingredient of the cell growth inhibitors of the present invention, as long as they bind to the antigen. Mouse antibodies, rat antibodies, rabbit antibodies, sheep antibodies, chimeric antibodies, humanized antibodies, and human antibodies may be used appropriately. Although the antibodies may be either polyclonal or monoclonal antibodies, monoclonal antibodies are preferred from the point of view that they can stably produce homogeneous antibodies. Polyclonal and monoclonal antibodies can be prepared by methods well known to those skilled in the art.

Hybridoma cells that produce monoclonal antibodies can basically be produced using conventional techniques, described as follows: Specifically, the hybridoma cells can be prepared by (1) conducting immunization using the desired antigen, or cells expressing the desired antigen, as the sensitizing antigen according to normal immunization methods; (2) fusing the obtained immunized cells with conventional parent cells by normal cell fusion methods; and (3) screening for monoclonal antibody-producing cells (hybridomas) using normal screening methods. Antigens can be prepared according to conventional methods such as methods using baculoviruses (*e.g.* WO 98/46777). Hybridomas can be produced, for example, according to the method of Milstein *et al.* (Kohler, G. and Milstein, C., Methods Enzymol. (1981) 73: 3-46). When the antigen has low immunogenicity, immunization can be performed by linking it to a macromolecule with immunogenicity, such as albumin.

Recombinant antibodies can also be used, and can be produced by (1) cloning an antibody gene from a hybridoma; (2) incorporating the antibody gene into an appropriate vector; (3) introducing the vector into a host; and (4) producing the recombinant antibodies by genetic engineering techniques (see, for example, Carl, A. K. Borrebaeck, James, W. Larrick, THERAPEUTIC MONOCLONAL ANTIBODIES, Published in the United Kingdom by MACMILLAN PUBLISHERS LTD, 1990). Specifically, cDNAs of the variable regions (V regions) of antibodies are synthesized from hybridoma mRNAs using reverse transcriptase. When DNAs encoding a V region of an antibody of interest are obtained, they are linked to DNAs encoding an antibody constant region (C region) of interest, and are then incorporated into expression vectors. Alternatively, DNAs encoding an antibody V region can be incorporated into expression vectors comprising DNAs of an antibody C region. The DNAs are incorporated into expression vectors such that expression is controlled by expression regulatory regions such as enhancers and promoters. Host cells are then transformed with these expression vectors to express the antibodies.

In the present invention, recombinant antibodies artificially modified to reduce heterologous antigenicity against humans can be used. Examples of such include chimeric antibodies and humanized antibodies. These modified antibodies can be produced using known methods. A chimeric antibody is an antibody comprising the antibody heavy chain and light chain variable regions of a nonhuman mammal such as a mouse, and the antibody heavy chain and light chain constant regions of a human. A chimeric antibody can be obtained by (1) ligating the DNA encoding a variable region of a mouse antibody to the DNA encoding a constant region of a human antibody; (2) incorporating them into an expression vector; and (3) introducing the vector into a host for production of the antibody.

A humanized antibody, which is also called a reshaped human antibody, is obtained by transplanting a complementarity determining region (CDR) of an antibody of a nonhuman mammal such as a mouse, into the CDR of a human antibody. Conventional genetic recombination techniques for the preparation of such antibodies are known. Specifically, a DNA sequence designed to ligate a CDR of a mouse antibody with the framework regions (FRs) of a human antibody is synthesized by PCR, using several oligonucleotides constructed to comprise overlapping portions at their ends. A humanized antibody can be obtained by (1) ligating the resulting DNA to a DNA which encodes a human antibody constant region; (2) incorporating this into an expression vector; and (3) transfecting the vector into a host to produce the antibody (see, European Patent Application No. EP 239, 400, and International Patent Application No. WO 96/02576). Human antibody FRs that are ligated via the CDR are selected where the CDR forms a favorable antigen-binding site. As necessary, amino acids in the framework region of an antibody variable region may be substituted such that the CDR of a reshaped human antibody forms an appropriate antigen-binding site (Sato, K. *et al.,* Cancer Res. (1993) 53, 851-856).

Methods for obtaining human antibodies are also known. For example, desired human antibodies with antigen-binding activity can be obtained by (1) sensitizing human lymphocytes with antigens of interest or cells expressing antigens of interest *in vitro;* and (2) fusing the sensitized lymphocytes with human myeloma cells such as U266 (see Examined Published Japanese Patent Application No. (JP-B) Hei 1-59878) . Alternatively, the desired human antibody can also be obtained by using the desired antigen to immunize a transgenic animal that comprises the entire repertoire of human antibody genes (see International Patent Application WO 93/12227, WO 92/03918, WO 94/02602, WO 94/25585, WO 96/34096, and WO 96/33735). Furthermore, techniques to obtain human antibodies by panning with a human antibody library are known. For example, the variable region of a human antibody is expressed as a single chain antibody (scFv) on the surface of a phage using phage display method, and phages that bind to the antigen can be selected. By analyzing the genes of selected phages, the DNA sequences encoding the variable regions of human antibodies that bind to the antigen can be determined. If the DNA sequences of scFvs that bind to the antigen are identified, appropriate expression vectors containing these sequences can be constructed, and human antibodies can be obtained. Such methods are already well known (see WO 92/01047, WO 92/20791, WO 93/06213, WO 93/11236, WO 93/19172, WO 95/01438, and WO 95/15388).

When the antibody genes have been isolated and introduced into an appropriate host, hosts and expression vectors can be used in appropriate combination to produce the antibodies. As eukaryotic host cells, animal cells, plant cells, and fungal cells may be used. Known animal cells include (1) mammalian cells such as CHO, COS, myeloma, baby hamster kidney (BHK), HeLa, and Vero cells; (2) amphibian cells such as Xenopus oocytes; or (3) insect cells such as sf9, sf21, and Tn5. Known plant cells include cells derived from the *Nicotiana* genus such as *Nicotiana tabacum,* which can be callus cultured. Known fungal cells include yeasts such as the *Saccharomyces* genus, for example *Saccharomyces cerevisiae,* and filamentous fungi such as the *Aspergillus* genus, for example *Aspergillus niger.* Prokaryotic cells can also be used in production systems that utilize bacterial cells. Known bacterial cells include *E. coli* and *Bacillus subtilis.* By transferring the antibody genes of interest into these cells using transformation, and then culturing the transformed cells *in vitro,* the antibodies can be obtained.

Furthermore, the antibody may be an antibody fragment or a modified antibody thereof, as long as it binds to PepT and inhibits its function. For example, the antibody fragment may be Fab, F (ab') 2, Fv, or single chain Fv (scFv) in which Fv from H or L chains are ligated by an appropriate linker. More specifically, the antibody fragment is obtained by (1) treating the antibody with enzymes such as papain and pepsin; (2) transferring it into an expression vector; and then (3) expressing it in an appropriate host cell (see, for example, Co, M. S. *et al.,* J. Immunol. (1994) 152, 2968-2976; Better, M. & Horwitz, A. H. Methods in Enzymology (1989) 178, 476-496, Academic Press, Inc.; Plueckthun, A. & Skerra, A. Methods in Enzymology (1989) 178, 476-496, Academic Press, Inc.; Lamoyi, E., Methods in Enzymology (1989) 121, 663-669; and Bird, R. E. *et al.,* TIBTECH (1991) 9, 132-137). scFv can be obtained by ligating the V regions of the antibody H-chain and L-chain. In the scFv, the V regions of the H chain and L chain are ligated via a linker, and preferably via a peptide linker (Huston, J. S. *et al.*, Proc. Natl. Acad. Sci. U.S.A (1988) 85, 5879-5883). The V regions of the scFv H chain and L chain may be derived from any of the antibodies described herein. The peptide linker used to ligate the V regions may be, for example, any single-chain peptide consisting of 12 to 19 residues. DNA encoding scFv can be amplified by PCR using as a template either the whole DNA, or a partial DNA encoding a desired DNA, selected from a DNA encoding the H chain or the V region of the H chain of the above antibody, and a DNA encoding the L chain or the V region of the L chain of the above antibody; and using a primer pair that defines the two ends. Further amplification can be subsequently conducted using the combination of DNA encoding the peptide linker portion, and the primer pair that defines both ends of the DNA to be ligated to the H chain and the L chain respectively. Once DNAs encoding scFvs are constructed, expression vectors containing the DNAs, and hosts transformed by these expression vectors, can be obtained according to conventional methods. Furthermore, scFvs can be obtained according to conventional methods using the resulting hosts. These antibody fragments can be produced in hosts by obtaining genes encoding the antibody fragments and expressing them in a manner similar to that outlined above. Antibodies bound to various types of molecules, such as polyethyleneglycol (PEG), may be used as modified antibodies. Such modified antibodies can be obtained by chemical modifications of the resulting antibodies. Methods for modifying antibodies are already established in the art. The term "antibody" in the present invention also encompasses the above-described antibodies.

Antibodies expressed and produced as described above can be purified by conventional methods for purifying normal proteins. Antibodies can be separated and purified by, for example, appropriately selecting and/or combining affinity columns such as a protein A column, or a chromatography column, filtration, ultrafiltration, salt precipitation, dialysis, and such (Antibodies A Laboratory Manual. Ed Harlow, David Lane, Cold Spring Harbor Laboratory, 1988).

Conventional means can be used to measure the antigen-binding activity of the antibodies (Antibodies A Laboratory Manual. Ed Harlow, David Lane, Cold Spring Harbor Laboratory, 1988). For example, enzyme linked immunosorbent assay (ELISA) , enzyme immunoassay (EIA), radioimmunoassay (RIA), or fluoroimmunoassay may be used.

Whether a particular molecule binds to a peptide transporter can be determined using conventional methods. Examples of conventional methods include immunoprecipitation, West-Western blotting, ELISA, EIA, RIA, fluoroimmunoassay, and methods using a biosensor utilizing surface plasmon resonance effect.

Candidate compounds for the cell growth inhibitors of the present invention can be screened using the activity of binding to this kind of peptide transporter as an indicator. Specifically, a test sample is contacted with a peptide transporter, binding between the peptide transporter and the test sample is detected, and compounds that bind to the peptide transporter can be selected. There are no particular limitations as to the test samples. Examples include cell extracts, cell culture supernatants, products of fermenting microorganisms, extracts from marine organisms, plant extracts, purified or crude purified proteins (including antibodies) , peptides, non-peptidic compounds, synthetic low-molecular-weight compounds, and natural compounds. The peptide transporter can be contacted with the test sample, for example, as a purified protein, in a form bound to a carrier, as a fusion protein with another protein, in a form expressed on a cell membrane, or as a membrane fraction. When selecting strong candidates for the cell growth inhibitors of this invention from compounds that bind to a peptide transporter obtained in this manner, it is useful to determine whether these compounds inhibit the transport function of the peptide transporter. Whether a particular molecule inhibits the transport function of a peptide transporter can be judged by conventional methods, for example by labeling a substrate such as a peptide with a radioactive substance (*e.g.* ¹⁴C), or a fluorescent substance, and then measuring the amount of the substrate incorporated into peptide transporter-expressing cells.

Substances that suppress the expression of peptide transporters can be used as active ingredients for the cell growth inhibitors of this invention. Examples of such substances include antisense oligonucleotides against peptide transporter genes. Examples of antisense oligonucleotides include an antisense oligonucleotide that hybridizes to any site of the DNA or mRNA encoding a peptide transporter. Preferably, the antisense oligonucleotide is against at least 15 or more continuous nucleotides in the DNA or mRNA of a peptide transporter. More preferably, the antisense oligonucleotide is against at least 15 or more continuous nucleotides comprising a translation initiation codon. Derivatives and modified forms of these can also be used as antisense oligonucleotides, including modified lower alkylphosphonates such as methyl phosphonate forms and ethyl phosphonate forms, modified phosphorothioate, or modified phosphoroamidate.

There are no particular limitations as to the cells to be targeted by the cell growth inhibitors of the present invention, but cancer cells such as pancreatic cancer cells, liver cancer cells, lung cancer cells, esophageal cancer cells, breast cancer cells, and colon cancer cells are preferred, and pancreatic cancer cells are especially preferred. The cell growth inhibitors of the present invention are used for the purpose of treatment and prevention of diseases caused by cell growth, and more specifically of cancers such as pancreatic cancer.

The cell growth inhibitors of the present invention can be administered either orally or parenterally, but are preferably administered parenterally. Specific examples include injections, nasal formulations, pulmonary formulations, and cutaneous formulations. For example, injections can be administered systemically or locally by intravenous injection, intramuscular injection, intraperitoneal injection, or subcutaneous injection. Furthermore, the method of administration can be selected appropriately according to the age and symptoms of the patient. A single dose can be selected, for example, from within the range of 0.0001 mg to 1,000 mg per kg body weight. Alternatively, the dose can be selected, for example, from within the range of 0.001 to 100,000 mg/body for each patient. However, the dose of a therapeutic agent of the present invention is not limited to these examples. Furthermore, the therapeutic agents of the present invention can be formulated according to standard methods (see, for example, Remington's Pharmaceutical Science, latest edition, Mark Publishing Company, Easton, U.S.A), and may comprise pharmaceutically acceptable carriers and additives.

### Brief Description of the Drawings

Fig. 1 shows the PepT1 inhibiting ability of AT-264 in Caco-2 cells.
Fig. 2 shows the cell growth inhibitory effect of AT-264 against human pancreatic cancer cell line AsPC-1. The data are shown as an average ± S.D. (n=3 to 4).
Fig. 3 shows the PepT2 inhibiting ability of AT-264 in BaF3/PepT2. The data are shown as an average ± S.D. (n=3 to 4).
Fig. 4 shows the cell growth inhibitory effect of AT-264 against human pancreatic cancer cell line BxPC-3. The data are shown as an average ± S.D. (n=6).

### Best Mode for Carrying out the Invention

Hereinafter, the present invention is specifically illustrated with reference to Examples, but it is not to be construed as being limited thereto.

### [Example 1] Inhibitory effect of AT-264 on PepT1 activity

The structure of AT-264 is represented by the structural formula below. The following experiments confirmed that this compound is an inhibitor of peptide transporters (PepTs).

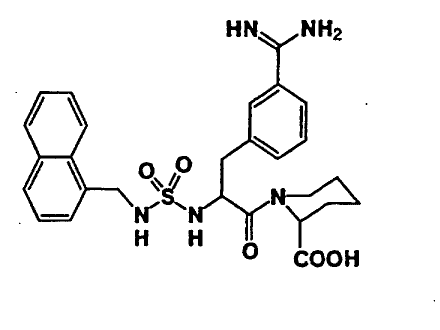

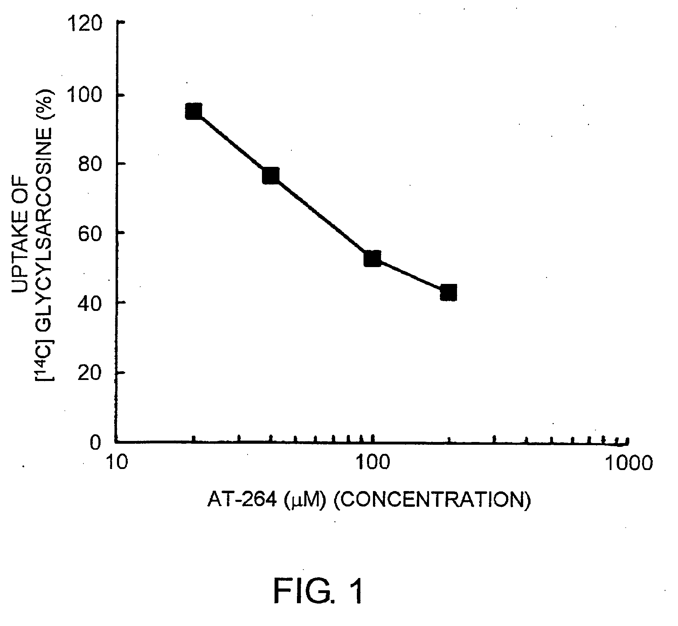

AT-264's ability to inhibit PepT was examined using a human colon cancer cell line (Caco-2 cells) . The results were that the IC₅₀ for uptake of radioactive substrate [¹⁴C] glycylsarcosine into cells was 100 µM (Fig. 1) . In Caco-2 cells, only PepT1 was expressed, and PepT1 function was considered to be inhibited by AT-264. The purity of AT-264 used in the present experiment was approximately 50% to 60%, and the actual IC₅₀ was thought to be about 50 µM or so.

### [Example 2] The cell growth inhibitory effect of AT-264 against human pancreatic cancer cell line AsPC-1

AT-264 was dissolved in RPMI1640 - 10 mM Hepes (hereinafter, abbreviated to 'the medium') containing 0.5% ethanol and 0.5% DMSO to prepare 2.5 mM AT-264 solution. Then, this solution was diluted with the medium to prepare 0.625 mM and 0.0625 mM AT-264 solutions.

A 5x10⁴ cells/mL solution of human pancreatic cancer cell line AsPC-1 was prepared using a medium containing 50% FBS. This suspension was plated onto a 96-well plate pre-coated with Collagen type I at 40 µL/well (2x10³ cells), and 160 µL of the AT-264 solution was added. This was cultured for six days in a CO₂ incubator (on the second day of culturing, 100 units/mL penicillin and 0.1 mg/mL streptomycin were added) . On the sixth day of culturing, the number of viable cells was quantified by MTS assay.

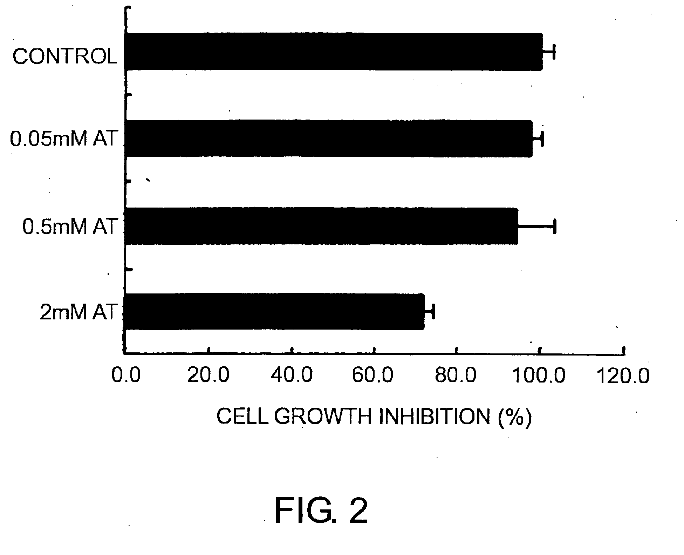

The results of the cell growth experiment are shown in Fig. 2. Cell growth inhibition was confirmed to be approximately 30% in the presence of 2 mM AT-264, and was present even in the presence of 0.5 mM AT-264, although slight. Morphological changes to AsPC-1 were not observed under the microscope, even in the presence of AT-264. Furthermore, the results of RT-PCR indicated that in AsPC-1, PepT1 expression was greater than PepT2. Accordingly, cell growth inhibition by AT-264 was considered to be due to the inhibition of PepT1 function, and not to non-specific cytotoxicity.

### [Example 3] Inhibitory effect of AT-264 on PepT2 activity

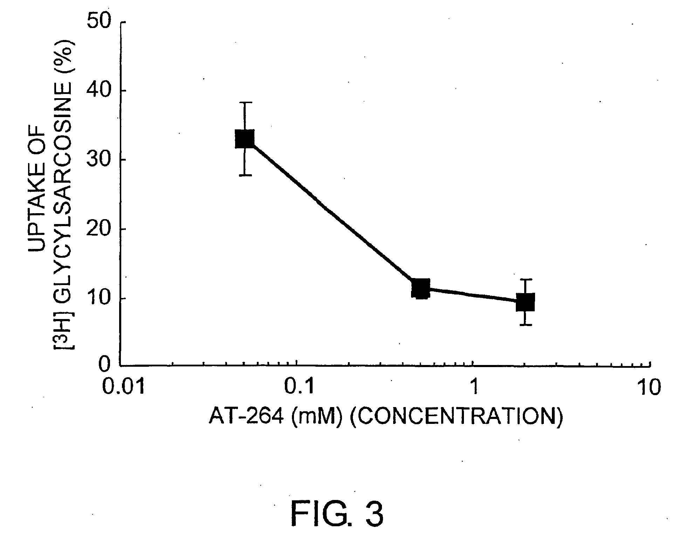

The ability of AT-264 to inhibit PepT2 was examined using a murine bone marrow-derived cell line BaF3 in which human PepT2 is forced to be expressed (hereinafter, abbreviated to BaF3/PepT2). As a result, the uptake of radioactive substrate [³H] glycylsarcosine into cells was inhibited in a concentration-dependent manner (Fig. 3) . Accordingly, AT-264 was found to inhibit the function of not only PepT1, but also of PepT2.

### [Example 4] Cell growth inhibitory effect of AT-264 on human pancreatic cancer cell line BxPC-3

AT-264 was dissolved in RPMI1640 - 10 mM Hepes with 100 units/mL penicillin and 0.1 mg/mL streptomycin (hereinafter, abbreviated to 'the medium') containing 0.5% ethanol and 0.5% DMSO to prepare 2.5 mM AT-264 solution. Furthermore, this solution was diluted with the medium to prepare 0.625 mM and 0.0625 mM AT-264 solutions.

5x10⁴ cells/mL solution of BxPC-3 was prepared using the medium containing 50% FBS. This suspension was plated onto a 96-well plate pre-coated with Collagen type I at 40 µL/well (2x10³ cells), and 160 µL of AT-264 solution was added. This was cultured for six days in a CO₂ incubator, and on the sixth day of culturing, the number of viable cells was quantified by MTS assay.

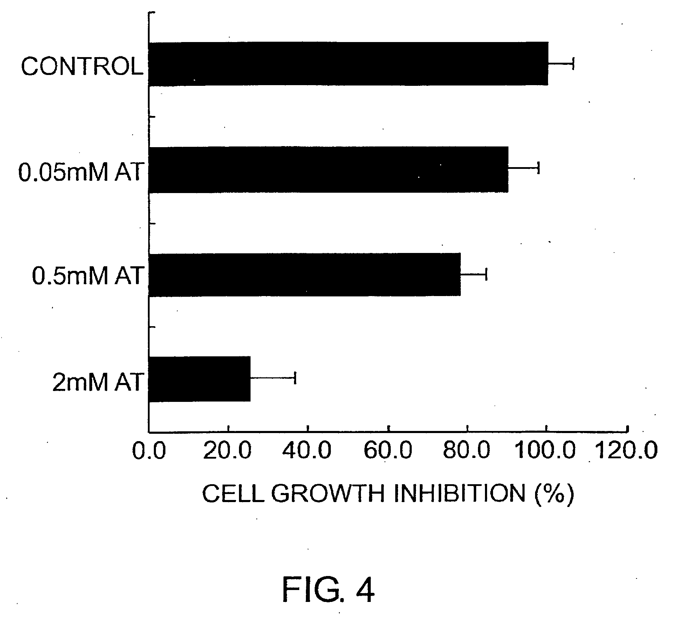

The results of the cell growth experiment are shown in Fig. 4. Cell growth inhibition was confirmed to be approximately 75% in the presence of 2 mM AT-264, and approximately 20% even in the presence of 0.5 mM AT-2 64. Morphological changes to BxPC-3 were not observed under the microscope, even in the presence of AT-264. Furthermore, the RT-PCR results showed that PepT2 expression was greater than PepT1 expression in BxPC-3. Accordingly, cell growth inhibition by AT-264 was considered to be due to the inhibition of PepT2 function, and not due to cytotoxicity.

### Industrial Applicability

The present invention revealed that cell growth can be suppressed by inhibiting the activity of peptide transporters. Accordingly, cell growth inhibitors can be developed using peptide transporters as targets. Such cell growth inhibitors are highly expected to be of use in suppressing the growth of cancers (for example, pancreatic cancer).

## Claims

1. A cell growth inhibitor comprising as an active ingredient a substance that inhibits a peptide transporter.

2. The cell growth inhibitor of claim 1, wherein the peptide transporter is a proton-driven peptide transporter.

3. The cell growth inhibitor of claim 1, wherein the peptide transporter is PepT1 or PepT2.

4. The cell growth inhibitor of any one of claims 1 to 3, wherein the peptide transporter inhibitory substance is a substance that inhibits the transport function of a peptide transporter by binding to the peptide transporter.

5. The cell growth inhibitor of any one of claims 1 to 3, wherein the peptide transporter inhibitory substance is a sulfamide derivative represented by the following formula (I): 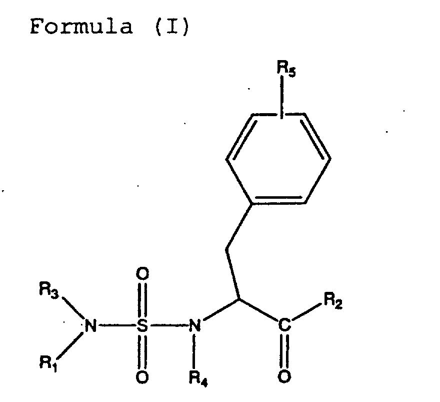 wherein,
R₁ represents a hydrogen atom, lower alkyl group, or amino protecting group;
R₂ represents a substituted or unsubstituted, fused or non-fused nitrogen atom-containing heterocycle;
R₃ represents a A-(CH₂)ₘ- group, a hydrogen atom, or a substituted or unsubstituted lower alkyl group, wherein, A represents a substituted or unsubstituted aryl group, substituted or unsubstituted, fused or non-fused heterocycle, or substituted or unsubstituted lower cycloalkyl group, and m represents an integer from 0 to 6, and the - (CH₂)ₘ- may be substituted with one or more substituents;
R₄ represents a hydrogen atom, lower alkyl group, or amino protecting group;
R₅ represents -C(=NR₆)NH₂, -NH-C(=NR₆)NH₂, or - (CH₂)ₙ-NHR₆ group,
wherein R₆ represents a hydrogen atom, lower alkyl group, hydroxyl group, acyl group, acyloxy group, lower alkoxy group, lower alkoxycarbonyl group, lower alkoxycarbonyloxy group, or lower hydroxyalkylcarbonyloxy group, n represents an integer from 0 to 2, and the -(CH₂)ₙ- may be substituted with one or more substituents.

6. The cell growth inhibitor of any one of claims 1 to 5, wherein the inhibitor suppresses the growth of a cancer cell.

7. The cell growth inhibitor of claim 6, wherein the cancer cell is a pancreatic cancer cell.
